# EUROPEAN PATENT SPECIFICATION

(11) **EP 4 057 845 B1**
(45) Date of publication and mention of the grant of the patent: **18.10.2023**
(21) Application number: 20800145.3
(22) Date of filing: 04.11.2020
(51) Int. Cl.: A24B 15/16, A24B 15/24, A24F 40/05, A61K 9/00, A61K 31/465, A61M 11/00

(54) **IMPROVED TOBACCO FLAVOURED DRY POWDER FORMULATION**
VERBESSERTE TROCKENPULVERFORMULIERUNG MIT TABAKAROMA
FORMULATION AMÉLIORÉE DE POUDRE SÈCHE AROMATISÉE AU TABAC

(30) Priority: 14.11.2019 EP 19209263
(43) Date of publication of application: 21.09.2022
(73) Proprietor: Philip Morris Products S.A., 2000 Neuchâtel (CH)
(72) Inventor: FLORACK, Dionisius, 2000 Neuchâtel (CH); LANASPÈZE, Sébastien, 2000 Neuchâtel (CH); SPADARO, Fabiana, 2000 Neuchâtel (CH)
(74) Representative: Civera, Andrea
(86) International application number: PCT/EP2020/080924
(87) International publication number: WO 2021/094160

(56) References cited:
- WO-A1-2018/197879
- WO-A1-2018/220475
- WO-A2-2015/166350
- US-A- 3 424 171
- US-A- 4 150 677
- US-A1- 2016 360 780

## Description

The present invention relates to a tobacco flavoured dry powder formulation for inhalation, which may find use as a component of a powder system that includes both particles comprising nicotine and particles containing flavour, such as for example one wherein the flavour particles are larger than the nicotine particles. Further, the present invention relates to a method of producing tobacco flavoured powder particles.

Dry powder inhalers (DPI) are known and are used to treat respiratory diseases by delivering a dry powder comprising a pharmaceutical in aerosol form through inhalation to a patient's airways. Typically, a DPI is a breath-actuated device that delivers the drug in the form of particles contained in a capsule or blister that is punctured prior to use. Since the drug is processed, weighed, and packed in powder form, decomposition, separation, and microbiological contamination hazards are minimal compared to wet formulations. Examples of powder inhalers are known from WO 2018/220475 A1 and WO 2015/166350 A2.

For delivery into the lungs, particles in the range of 1 to 5 micrometres are preferred. In pharmaceutical dry powders, the active pharmaceutical ingredient (API) may be agglomerated on the surface of larger carrier particles, such as lactose. Pharmaceutical dry powders containing lactose as a carrier can be in the range of 20 to 100 micrometres. DPIs operate complex mechanisms to ensure such agglomerates disperse, break up or disaggregate before the API can be inhaled into the lungs.

DPIs rely on the force of the patient's inhalation to entrain the powder from an inhalation device to subsequently break up the powder into particles that are small enough to enter the lungs. Sufficiently high inhalation rates are required to ascertain correct dosing and complete disaggregation of the powder. Typically, a large amount of API remains attached on the surface of the carrier and is deposited in the upper airways due to incomplete de-aggregation of the powder. Inhalation rates of existing DPIs are usually in the range of 20-100 litres/min (L/min). Existing DPIs are therefore only suitable for delivering dry powders to users in a manner that is different from the inhalation rate associated with smoking articles.

Thus, existing DPIs are generally not suitable to deliver dry powder particles to the lungs in a manner consistent with conventional smoking regimes. For example, DPIs often strive to provide an entire dry powder dose in a single breath. In contrast, conventional smoking regimes involve a number of comfortable puffs.

A solution addressing this issue has been proposed, for example, in WO 2019/003118, which describes a container or capsule, a powder system and an inhaler article adapted to provide particles to the lungs at inhalation or air flow rates that are within conventional smoking regime inhalation or air flow rates. A consumer may take a plurality of inhalations or "puffs" where each "puff" delivers a uniform fractional amount of dry powder contained within a container or capsule contained within the capsule cavity of the inhaler article described in WO 2019/003118. The inhaler article may have a form similar to a conventional cigarette and may mimic the ritual of conventional smoking and may provide a pleasure or entertainment form of nicotine delivery. In some embodiments, the inhaler article is adapted to deliver a powder system comprising a first plurality of particles and a second plurality of particles. The first plurality of particles have a particle size that is larger than the particle size of the second plurality of particles. The first plurality of particles may be free of nicotine and include a flavour component, and are preferably free-flowing. The second plurality of particles comprise nicotine and is preferably free-flowing.

A process is known from US 6056949 for the preparation of a substantially spherical, practically dust-free aromatic and odoriferous granulated material which is free-flowing, mechanically stable, and has a narrow grain size distribution. According to US 6056949, any conventional flavourant or odorant may be used in the manufacture of such dry powder, including fruit, such as citrus, berry, tobacco, flowers, wood, spice, and amber. Powder particles obtained by the process of US 6056949 are described as having a size from 0.2 millimetres to 1 millimetre.

WO 2018/197879 A1 discloses a method of extracting one or more volatile compounds of interest from tobacco material, the method comprising the steps of: i) providing tobacco material; ii) subjecting the tobacco material to steam distillation; and iii) extracting one or more volatile compounds of interest from the tobacco material with a solvent; wherein distillation step (ii) and extraction step (iii) are carried out simultaneously and at a pH of no greater than 2, and wherein the period during which both the distillation step (ii) and the extraction step (iii) are carried out is from about 8 to about 20 hours.

Other examples of methods for extracting a flavour constituent from a tobacco raw material for use in tobacco products are known from US 2016/360780 A1, US 3 424 171 A, and US 4 150 677 A.

It would be desirable to provide such an improved tobacco flavoured dry powder that can be readily used in an inhaler device associated with a conventional smoking regime or in the manufacture of a powder system for use in one such inhaler device.

Equally, it would be desirable to provide a method for the manufacture of such an improved tobacco flavoured dry powder, particularly one that can be carried out efficiently using existing apparatus and techniques.

The present disclosure relates to a tobacco flavoured dry powder formulation comprising a plurality of particles. The particles comprise a base material and a tobacco flavouring composition. A first ratio by weight of (β-ionone + β-damascenone) to (phenol) in the tobacco flavoured dry powder formulation is greater than 0.25.

Further, the present disclosure relates to a method of producing a tobacco flavoured powder formulation. The method comprises a step of preparing a tobacco starting material. The method comprises a step of heating the tobacco starting material at an extraction temperature of between 100 degrees Celsius and 160 degrees Celsius for at least 90 minutes. The method comprises a step of collecting the volatile compounds released from the tobacco starting material during the heating step. The method comprises a step of forming a liquid tobacco flavouring composition comprising the collected volatile compounds. The method comprises a step of combining a base material and the liquid tobacco flavouring composition to form tobacco flavoured particles, wherein in the step of preparing the tobacco starting material, the tobacco starting material is not subjected to any treatment adapted to alter the pH of the tobacco.

In addition, the present disclosure relates to a powder system comprising a first plurality of particles and a second plurality of particles. The first plurality of particles has a particle size of at least about 20 micrometres. The second plurality of particles has a particle size of about 10 micrometres or less. The first plurality of particles comprises a base material and a tobacco flavouring composition. A first ratio by weight of (β-ionone + β-damascenone) to (phenol) in the tobacco flavouring composition is greater than 0.25. The second plurality of particles comprises nicotine. Further, the second plurality of particles may comprise a sugar and ar amino acid.

Further, the present disclosure relates to a powder system comprising a first plurality of tobacco flavoured particles. The tobacco flavoured particles may have a particle size of at least about 20 micrometres. The powder system may comprise a second plurality of particles. The second plurality of particles may have a particle size of less than about 20 micrometres. A first ratio by weight of (β-ionone + β-damascenone) to (phenol) in the tobacco flavoured particles of the first plurality may be greater than 0.25.

According to the present invention there is provided a tobacco flavoured dry powder formulation comprising a plurality of particles comprising a base material and a tobacco flavouring composition. A first ratio by weight of (β-ionone + β-damascenone) to (phenol) in the tobacco flavoured dry powder formulation is greater than 0.25.

According to the present invention there is also provided a method of producing a tobacco flavoured powder formulation. The method comprises a first step of preparing a tobacco starting material. The method comprises a second step of heating the tobacco starting material at an extraction temperature of between 100 degrees Celsius and 160 degrees Celsius for at least 90 minutes. The method comprises a third step of collecting the volatile compounds released from the tobacco starting material during the heating step. The method comprises a fourth step of forming a liquid tobacco flavouring composition comprising the collected volatile compounds. The method comprises a fifth step of combining a base material and the liquid tobacco flavouring composition to form tobacco flavoured particles.

According to the present invention there is further provided a powder system comprising a first plurality of particles and a second plurality of particles. The first plurality of particles have a particle size of at least about 20 micrometres. The second plurality of particles have a particle size of about 10 micrometres or less. The first plurality of particles comprise a base material and a tobacco flavouring composition. A first ratio by weight of (β-ionone + β-damascenone) to (phenol) in the tobacco flavouring composition is greater than 0.25.. The second plurality of particles comprise nicotine.

According to the present invention there is also provided a powder system comprising: a first plurality of tobacco flavoured particles having a particle size of at least about 20 micrometres and a second plurality of particles having a particle size of less than about 20 micrometres, wherein a first ratio by weight of (β-ionone + β-damascenone) to (phenol) in the tobacco flavoured particles of the first plurality is greater than 0.25.

It will be appreciated that any features described below with reference to the tobacco flavoured dry powder formulation of the present invention or to the method of producing a tobacco flavoured dry powder formulation of the present invention or to the powder system of the present invention are equally applicable to any other of the powder formulation, method, and powder system.

As used herein with reference to the present invention, the term "dry powder formulation" denotes a formulation that contains finely dispersed solid particles having a certain particle size distribution that are capable of being readily dispersed in or by means of an inhaler and be administered to a subject via inhalation so that a portion of the particles reach a tissue of the oral cavity or of the upper respiratory tract, such as for example the pharynx or the throat in general. Depending on the size of the particles, which is defined by their aerodynamic diameters, the particles of a dry powder formulation may additionally be suitable for pulmonary administration.

The size of a particle, as stated herein, preferably refers to the aerodynamic diameter of the particle. The aerodynamic diameter of a particle is defined as that of a sphere having a density of 1 gram per cubic centimetre, which settles in still air at the same velocity as the particle in question.

In particular, for a powder system reference is commonly made to the mass median aerodynamic diameter (MMAD), one of the metrics most widely adopted as a single number descriptor of aerodynamic particle-size distribution. The MMAD is a statistically derived figure for a particle sample: by way of example, an MMAD of 5 micrometres means that 50 percent of the total sample mass will be present in particles having aerodynamic diameters of less than 5 micrometres, and that the remaining 50 percent of the total sample mass will be present in particles having an aerodynamic diameter greater than 5 micrometres. In the context of the present invention, when describing a powder system, the term "particle size" preferably refers to the MMAD of the powder system.

The MMAD of a powder system is preferably measured with a cascade impactor. Cascade impactors are instruments which have been extensively used for sampling and separating airborne particles for determining the aerodynamic size classification of aerosol particles. In practice, cascade impactors separate an incoming sample into discrete fractions on the basis of particle inertia, which is a function of particle size, density and velocity. A cascade impactor typically comprises a series of stages, each of which comprises a plate with a specific nozzle arrangement and a collection surface. As nozzle size and total nozzle area both decrease with increasing stage number, the velocity of the sample-laden air increases as it proceeds through the instrument. At each stage, particles with sufficient inertia break free from the prevailing air stream to impact on the collection surface. Therefore, at any given flow rate, each stage is associated with a cut-off diameter, a figure that defines the size of particles collected. With increasing stage number, velocity increases and so stage cut-off diameter decreases. Thus, the cut-off diameter associated with a given stage is a function of the air-flow rate used for testing. To reflect in-use performance, nebulisers are routinely tested at 15 L/min and dry powder inhalers may be tested at flow rates up to 100 L/min.

Preferably, in the context of the present invention, the MMAD of a powder system is measured with a Next Generation Impactor (NGI) 170 (available from Copley Scientific AG). The NGI is a high performance, precision, particle classifying cascade impactor having seven stages plus a Micro-Orifice Collector (MOC). Characteristics and operation principle of a NGI are described, for example, in Marple et al., Journal of Aerosol Medicine - Volume 16, Number 3 (2003). More preferably, measurements are carried out at 20 ±3 degrees Celsius and relative humidity of 35 ± 5 percent.

A dry powder formulation typically contains less than or equal to about 15 percent by weight moisture, preferably less than or equal to about 10 percent moisture, even more preferably less than or equal to about 6 percent by weight moisture. Most preferably a dry powder formulation contains less than or equal to about 5 percent by weight moisture or even less than or equal to about 3 percent by weight moisture or even less than or equal to about 1 percent by weight moisture.

In some embodiments, the dry powder formulation may contain from about 1 percent by weight moisture to about 15 percent by weight moisture, preferably from about 3 percent by weight moisture to about 15 percent by weight moisture, even more preferably from about 5 percent by weight moisture to about 15 percent by weight moisture. In other embodiments, the dry powder formulation may contain from about 1 percent by weight moisture to about 10 percent by weight moisture, preferably from about 3 percent by weight moisture to about 10 percent by weight moisture, even more preferably from about 5 percent by weight moisture to about 10 percent by weight moisture. In further embodiments, the dry powder formulation may contain from about 1 percent by weight moisture to about 10 percent by weight moisture, preferably from about 3 percent by weight moisture to about 10 percent by weight moisture, even more preferably from about 5 percent by weight moisture to about 10 percent by weight moisture.

In some particularly preferred embodiments, the dry powder formulation may contain from about 1 percent by weight moisture to about 6 percent by weight moisture or from about 3 percent by weight moisture to about 6 percent by weight moisture or from about 5 percent by weight moisture to about 6 percent by weight moisture.

The particles may be micro-sized or nano-sized. The particles may have a narrow particle size distribution.

The term "micro-sized" is used herein with reference to the particles of a formulation in accordance with the present invention to refer broadly to particles having an average particle size of from about 1 micrometres to about 10 micrometres. The particle size may refer to the diameter of the particles where they are substantially spherical. The particles may be non-spherical and the particle size may refer to an equivalent diameter of the particles relative to spherical particles.

The term "nano-sized" is used herein with reference to the particles of a formulation in accordance with the present invention to refer broadly to particles having an average particle size of less than about 1000 nanometres, particularly between about 50 nanometres and about 1000 nanometres.

In the context of the present invention, the term "narrow particle size distribution" is used to indicate that a span value of the particles of a formulation in accordance with the invention is less than about 2. The span value is defined as Span=([particle diameter at 90 percent cumulative size]-[particle diameter at 10 percent cumulative size])/[particle diameter at 50 percent cumulative size], or defined arithmetically as (D90-D10)/D50.

As described briefly above, in contrast with existing dry powder formulations, a tobacco flavoured dry powder formulation in accordance with the present invention comprises a plurality of particles comprising a base material and a tobacco flavouring composition, a first ratio by weight of (β-ionone + β-damascenone) to (phenol) in the tobacco flavoured dry powder formulation being greater than 0.25.

Thus, the invention advantageously provides a tobacco flavoured dry powder formulation that may maximise the content of tobacco-flavour-related compounds whilst at the same time reducing the content of less desirable natural tobacco-derived compounds, such as furans and TSNAs. Further, the inventors have found that a tobacco flavoured dry powder formulation in accordance with the present invention is closer in flavour to natural tobacco compared with powder formulations obtained from artificial blends including synthetic compounds.

In addition, in preferred embodiments that will be described in detail below, it is advantageously possible to reduce mouth harshness and control the level of nicotine in the tobacco flavoured dry powder formulation.

As described briefly above, a tobacco flavoured dry powder formulation can be obtained by a method comprising a first step of preparing a tobacco starting material. Preferably, the tobacco starting material is a natural tobacco material.

As used herein with reference to the present invention, the term "natural tobacco material" describes any part of any plant member of the genus *Nicotiana,* including, but not limited to, leaves, midribs, stems and stalks. In particular, the natural tobacco material may comprise flue-cured tobacco material, Burley tobacco material, Oriental tobacco material, Maryland tobacco material, dark tobacco material, dark-fired tobacco material, Rustica tobacco material, as well as material from other rare or specialty tobaccos, or blends thereof. As will be described in more detail below, the tobacco material may be whole (for example, whole tobacco leaves), shredded, cut, ground, or aged. In some embodiments, the tobacco material may be a combination of one or more of whole shredded, cut, ground, and aged.

As used herein with reference to the method of the present invention, the term "liquid tobacco flavouring composition" describes the direct product of an extraction process carried out on a tobacco starting material. Thus, the tobacco extract typically includes a mixture of natural components separated from, removed from, or derived from, a natural tobacco material using tobacco extraction processing conditions and techniques. Thus, in one such process extracted tobacco components are removed from the natural tobacco material and separated from unextracted tobacco components.

Several methods are known for manufacturing a liquid tobacco extract usable as a liquid tobacco flavouring composition. By way of example, WO 2017/144705 discloses a method wherein a tobacco material is heated to a temperature between 50 and 250 degrees Celsius, and volatile species released from the heated tobacco material are collected to manufacture a liquid formulation (also referred to as an e-liquid) for use in an e-vaping device.

Maceration methods are also known, wherein a tobacco material is kept in suspension in an extraction liquid for a period of up to several weeks or even months. The resulting slurry is subsequently filtered, and the liquid phase thus collected can be used to manufacture a vaporisable liquid formulation. In one such method - so-called "cold maceration method" - there is generally no way of controlling the extraction conditions (e.g. temperature and pressure). In a variant of a maceration method, which has been described for example in US 2012/192880, the slurry is heated to 100 degrees Celsius or more.

The liquid phase collected upon filtration of the slurry, which represents the primary product of a maceration process, is highly diluted, and tends to have a low content of apolar tobacco flavour species. Additionally, the liquid phase typically contains little to no nicotine. As such, liquid extracts obtained by a maceration method generally need to be supplemented with additional ingredients, such as nicotine salts and glycerin, before being used in a vaporisable liquid formulation.

Alternative processes are known, wherein a tobacco material is substantially boiled in water for a period of hours or even days to form a vapour phase, and a distillate obtained by condensation of the vapour phase is continuously collected in a vessel. Over time, an oily, waxy layer containing a high proportion of apolar compounds builds up on the surface of the distillate.

On the one hand, the aqueous portion, above which the waxy layer builds up, and which contains nicotine and other water-soluble compounds, is recycled to the boiler. An apolar cosolvent may optionally be fed into the boiler with the aqueous portion in order to increase the extraction yield. On the other hand, the waxy phase is collected and ultimately forms the primary product of one such hydro-distillation process. Such product is often referred to as "tobacco essential oil", and contains a high proportion of apolar compounds found in tobacco, such as fatty acids, neophytadiene, etc. The tobacco essential oil obtained by one such method typically contains no nicotine. It is also known to subject tobacco material to an extraction process involving use of a volatile apolar solvent. Examples of suitable solvents are cyclic or acyclic short alkanes, as well as chlorinated solvents like dichloromethane. In one such process, the excess solvent may be evaporated by controlled heating under vacuum. Typically, this is done in the presence of ethanol, which has a higher boiling point than the extraction solvent, such that even traces of the extraction solvent can be detected.

The primary product of one such solvent-aided extraction process is often referred to as "tobacco absolute", and may contain traces of ethanol. It is a waxy product and contains a highly concentrated mixture of most of the apolar compounds that can be extracted with the specific solvent, generally including nicotine, which is generally present at relatively high concentrations.

An alternative extraction process involves contacting a tobacco material with a solvent under supercritical conditions, such as supercritical carbon dioxide. One such process is disclosed in US 2013/160777, and relies on the principle that volatile substances within a feed material contacted with a supercritical fluid may partition into the supercritical phase. After dissolution of any soluble material, the supercritical fluid containing the dissolved substances can be removed, and the dissolved components of the feed matter can be separated out from the supercritical fluid. The primary product of a supercritical extraction process is substantially similar to the "tobacco absolute" of a solvent-aided extraction process run at lower temperature and pressure, contains no residual solvent and typically has a high level of the waxy, apolar compounds and includes nicotine, which is generally present at relatively high concentrations.

However, all the tobacco extracts obtainable by methods known in the art tend to have a very low level - if any - of compounds associated with the flavour of heated tobacco, such as furaneol.

According to the present invention, the extraction steps for producing the liquid tobacco flavouring composition comprise heating the tobacco starting material under specific heating conditions, and collecting the volatile compounds generated. Such step of heating the natural tobacco material may comprise heating the natural tobacco material in a flow of inert gas or in a flow of a combination of an inert gas with water or steam. As an alternative, the step of heating the natural tobacco material may comprise heating the natural tobacco material under vacuum.

The liquid tobacco flavouring composition therefore consists of the mixture of natural tobacco components that have derived from the tobacco starting material and have been extracted or formed during the extraction process, typically in combination with one or more materials other than the tobacco starting material, such as a non-aqueous extraction solvent used during the extraction process.

As will be described in more detail below, the volatile compounds released from the starting tobacco material may be collected using a condensation technique wherein the volatile compounds are removed from a gaseous stream by saturating the volatile compounds in the gaseous stream. By way of example, an inert gas flow containing the volatile compounds may be directed into a conventional shell-and-tube condenser, which may be either water-cooled or air-cooled. As the extraction is typically carried out at an extraction temperature of between 100 degrees Celsius and 160 degrees Celsius, as described in more detail below, even inducing a small reduction of the temperature of the gaseous stream containing the volatile compounds by contacting the gaseous stream with ambient air may be enough to cause condensation of the volatile compounds.

As used herein with reference to the present invention, the term "aerosol former" refers to a compound or mixture of compounds that, in use, facilitates formation of an aerosol, and that preferably is substantially resistant to thermal degradation at the operating temperature of the aerosol-generating article or device. Examples of suitable aerosol-formers include: polyhydric alcohols, such as propylene glycol, triethylene glycol, 1,3-butanediol and glycerin; esters of polyhydric alcohols, such as glycerol mono-, di- or triacetate; and aliphatic esters of mono-, di- or polycarboxylic acids, such as dimethyl dodecanedioate and dimethyl tetradecanedioate.

The method of the present invention uses an extraction temperature within a specific range in combination with a specifically defined heating duration that advantageously provides an improved liquid tobacco flavouring composition having a significantly improved balance of desirable compounds to undesirable compounds. In particular, the extraction conditions of the method of the present invention provides a liquid tobacco flavouring composition having a maximised ratio of desirable compounds to undesirable compounds for the tobacco starting material. For example, the use of the specific combination of extraction temperature and time as defined enables the levels of undesirable compounds such as furans, carbonyls, phenols and TSNAs to be minimised.

The method of the present invention enables a liquid tobacco flavouring composition to be produced which has the desired levels of tobacco flavour compounds without the need for addition of such compounds after extraction.

In particular, the inventors have found that, in contrast to existing extraction processes such as the ones that have been discussed above, in methods in accordance with the present invention a liquid tobacco flavouring composition is advantageously provided that has a significantly higher content of compounds associated with the flavour of heated tobacco, such as for example furaneol. These compounds are substantially absent, or are present in trace amounts, in a liquid tobacco flavouring composition obtained by a maceration process, which also typically contains little to no nicotine. These compounds are also generally absent or present in trace amounts in a liquid tobacco flavouring composition obtained using a solvent, including under supercritical conditions. Similarly, a tobacco essential oil obtained by way of a distillation process also typically has a very low content - if any - of such compounds associated with the flavour of heated tobacco. According to the method of the present invention, the liquid tobacco flavouring composition obtained by the extraction steps is combined with a base material to form tobacco flavoured particles that advantageously have a significantly improved balance of desirable compounds to undesirable compounds.

As discussed above, liquid tobacco flavouring compositions obtained and used in a method in accordance with the invention present significant compositional differences with respect to tobacco extracts or liquid tobacco flavouring compositions obtained by the existing extraction processes. As such, they can be combined with a base material to form tobacco flavoured particles that have a distinct composition and flavour characteristics compared with currently available tobacco flavoured particles. In particular, liquid tobacco flavouring compositions obtained and used in a method in accordance with the invention may be used to provide tobacco flavoured particles that provide a tobacco taste which more closely resembles the taste of an aerosol generated by conventional cigarettes or upon heating tobacco in a heat-not-burn device with respect to tobacco flavoured particles produced from existing liquid tobacco flavouring compositions.

The method of producing a tobacco flavoured dry powder formulation of the present invention can be used effectively with all types and grades of tobacco as the starting tobacco material, including Burley tobacco, flue-cured tobacco and Oriental tobacco. The extraction steps of the method can be readily adjusted in order to provide a consistent liquid tobacco flavouring composition for a variety of blends of tobacco type. The method is additionally suitable for a variety of forms of tobacco starting material.

In many cases, the tobacco starting material can be heated without the need for significant pre-treatment steps. The method can therefore be carried out efficiently. The method can advantageously be carried out using existing apparatus and techniques, which can be readily modified in order to carry out the method steps of the present invention.

In a tobacco flavoured dry powder formulation in accordance with the invention, the ratio by weight of (β-ionone + β-damascenone) to (phenol) is greater than 0.25. This may be achieved for example by combining a base material with a tobacco flavouring composition having a ratio by weight of (β-ionone + β-damascenone) to (phenol) greater than 0.25. Such ratio is higher when the amount of desirable flavourant compounds β-ionone and β-damascenone is higher, or when the amount of phenol is lower.

Preferably, the ratio by weight of (β-ionone + β-damascenone) to (phenol) is greater than 0.5. More preferably, the ratio by weight of (β-ionone + β-damascenone) to (phenol) is greater than 1. Even more preferably, the ratio by weight of (β-ionone + β-damascenone) to (phenol) is greater than 1.5. Most preferably, the ratio by weight of (β-ionone + β-damascenone) to (phenol) is greater than 2.

In tobacco flavoured dry powder formulations in accordance with the present invention the ratio by weight of (β-ionone + β-damascenone) to (phenol) is preferably less than or equal to about 10. More preferably, the ratio by weight of (β-ionone + β-damascenone) to (phenol) is less than or equal to 5.

In some embodiments, the ratio by weight of (β-ionone + β-damascenone) to (phenol) is from about 0.25 to about 10, more preferably from about 0.5 to about 10, even more preferably from about 1 to about 10, particularly preferably from about 1.5 to about 10, most preferably from about 2 to about 10. In other embodiments, the ratio by weight of (β-ionone + β-damascenone) to (phenol) is from about 0.25 to about 5, more preferably from about 0.5 to about 5, even more preferably from about 1 to about 5, particularly preferably from about 1.5 to about 5, most preferably from about 2 to about 5.

Particles having a ratio by weight of (β-ionone + β-damascenone) to (phenol) in the ranges described above may be obtained by combining a base material with a tobacco flavouring composition wherein a ratio by weight of ((β-ionone + β-damascenone) to (phenol) falls in the ranges described above.

In a tobacco flavoured dry powder formulation in accordance with the invention, a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) may be greater than 0.2. Preferably, a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) is at least about 0.5. The above ratio is higher when the amount of desirable flavourant compounds β-ionone and β-damascenone is higher, or when the amount of TSNAs and 2-furanemethanol is lower.

More preferably, in a tobacco flavoured dry powder formulation in accordance with the present invention the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) is greater than 1.

In preferred embodiments, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) is greater than 1.5.

By way of example, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) may be between about 1 and about 10 or between about 1.5 and about 6. In particularly preferred embodiments, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) is from about 2 to about 4.

Particles having a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the ranges described above may be obtained by combining a base material with a tobacco flavouring composition wherein a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) falls in the ranges described above.

Particles of a tobacco flavoured dry powder formulation in accordance with the present invention may further comprise other desirable compounds derived directly from natural tobacco, many of which are flavourants. By way of example, the tobacco flavoured dry powder formulation may comprise one or more of furaneol, 2,3-diethyl-5-methylpyrazine, acetic acid, vanillin, 2-ethyl-3,5-dimethylpyrazine, 2-methylbutanoic acid, 3-methylbutanoic acid, 3-methyl-2,4-nonanedione, 2-methoxyphenol, 2-phenylethanol, eugenol and sotolone.

The particles of a dry tobacco flavoured powder formulation in accordance with the invention comprise β-ionone. The dry tobacco flavoured powder formulation may comprise at least 0.100 micrograms β-ionone per gram of the dry tobacco flavoured powder formulation, preferably at least 0.200 micrograms β-ionone per gram of the dry tobacco flavoured powder formulation, more preferably at least 0.300 micrograms β-ionone per gram of the dry tobacco flavoured powder formulation, most preferably at least 0.400 micrograms β-ionone per gram of the dry tobacco flavoured powder formulation. In preferred embodiments, the dry tobacco flavoured powder formulation comprises at least 0.500 micrograms β-ionone per gram of the dry tobacco flavoured powder formulation, more preferably at least 0.600 micrograms β-ionone per gram of the dry tobacco flavoured powder formulation, even more preferably at least 0.700 micrograms β-ionone per gram of the tobacco flavouring composition, most preferably at least 0.800 micrograms β-ionone per gram of the dry tobacco flavoured powder formulation. In particularly preferred embodiments, the dry tobacco flavoured powder formulation comprises at least 0.9 micrograms β-ionone per gram of the dry tobacco flavoured powder formulation, preferably at least 1.00 micrograms β-ionone per gram of the dry tobacco flavoured powder formulation, more preferably at least 1.10 micrograms β-ionone per gram of the dry tobacco flavoured powder formulation, even more preferably at least 1.20 micrograms β-ionone per gram of the dry tobacco flavoured powder formulation, most preferably at least 1.30 micrograms β-ionone per gram of the dry tobacco flavoured powder formulation.

The ratio by weight of (β-ionone) to (phenol) in a tobacco flavoured dry powder formulation in accordance with the present invention may be at least about 0.150, for example at least about 0.200, preferably at least about 0.400, more preferably at least about 0.600, most preferably at least about 0.800, such as at least about 1.200.

Particles having a ratio by weight of (β-ionone) to (phenol) in the ranges described above may be obtained by combining a base material with a tobacco flavouring composition wherein a ratio by weight of (β-ionone) to (phenol) falls in the ranges described above.

The ratio by weight of (β-ionone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600) in a tobacco flavoured dry powder formulation in accordance with the present invention may be at least about 0.300, for example at least about 0.500, preferably at least about 0.750, more preferably at least about 1.00, most preferably at least about 1.20, such as at least about 1.80.

Particles having a ratio by weight of (β-ionone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600) in the ranges described above may be obtained by combining a base material with a tobacco flavouring composition wherein a ratio by weight of (β-ionone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600) falls in the ranges described above.

The particles of the tobacco flavoured dry powder formulation comprise β-damascenone. The tobacco flavoured dry powder formulation may comprise at least 0.100 micrograms β-damascenone per gram of the tobacco flavoured dry powder formulation, preferably at least 0.350 micrograms β-damascenone per gram of the tobacco flavoured dry powder formulation, more preferably at least 0.600 micrograms β-damascenone per gram of the tobacco flavoured dry powder formulation, most preferably at least 0.850 micrograms β-damascenone per gram of the tobacco flavoured dry powder formulation. In preferred embodiments, the tobacco flavoured dry powder formulation comprises at least 1.10 micrograms β-damascenone per gram of the tobacco flavoured dry powder formulation, more preferably at least 1.35 micrograms β-damascenone per gram of the tobacco flavoured dry powder formulation, even more preferably at least 1.60 micrograms β-damascenone per gram of the tobacco flavoured dry powder formulation, most preferably at least 1.85 micrograms β-damascenone per gram of the tobacco flavoured dry powder formulation. In particularly preferred embodiments, the tobacco flavoured dry powder formulation comprises at least 2.10 micrograms β-damascenone per gram of the tobacco flavoured dry powder formulation, preferably at least 2.35 micrograms β-damascenone per gram of the tobacco flavoured dry powder formulation, more preferably at least 2.60 micrograms β-damascenone per gram of the tobacco flavoured dry powder formulation, even more preferably at least 2.75 micrograms β-damascenone per gram of the tobacco flavoured dry powder formulation, most preferably at least 2.90 micrograms β-damascenone per gram of the tobacco flavoured dry powder formulation.

In some embodiments, the tobacco flavouring composition combined with the base material to form the particles of a tobacco flavoured dry powder formulation in accordance with the invention may comprise a non-aqueous solvent. This may be the case, for example, if a non-aqueous solvent has been used during the extraction steps for collecting the volatile compounds released upon heating the tobacco starting material. The non-aqueous solvent may be an aerosol former. Thus, a tobacco flavoured powder formulation in accordance with the present invention may comprise a non-aqueous solvent, preferably a non-aqueous solvent that is an aerosol former.

In those embodiments, the non-aqueous solvent may be one or more of glycerin, propylene glycol, triacetin, and 1,3-propanediol.

In preferred embodiments, the tobacco flavoured dry powder formulation comprises less than 5 percent by weight of a non-aqueous solvent. More preferably, the tobacco flavoured dry powder formulation comprises less than 3 percent by weight of a non-aqueous solvent. Even more preferably, the tobacco flavoured dry powder formulation comprises less than 1 percent by weight of a non-aqueous solvent. In some particularly preferred embodiments, the tobacco flavoured dry powder formulation substantially does not contain any non-aqueous solvent.

In some embodiments, the tobacco flavoured dry powder formulation may further comprise one or more water-soluble organic acids. As used herein with reference to the invention, the term "water-soluble organic acid" describes an organic acid having a water solubility at 20 degrees Celsius of greater than or equal to about 500 mg/ml.

Without wishing to be bound by theory, it is understood that some amount of a water-soluble organic acid may be extracted from the starting tobacco material and end up in the flavouring composition which is combined with the base material to form the flavour powder particles.

In some embodiments, the water-soluble organic acid is acetic acid.

Typically, the particles of a tobacco flavoured dry powder formulation in accordance with the present invention may comprise at least about 0.001 percent by weight of nicotine based on the weight of the tobacco flavoured dry powder formulation.

The particles of a tobacco flavoured dry powder formulation in accordance with the present invention preferably comprise less than or equal to about 5 percent by weight of nicotine based on the weight of the tobacco flavoured dry powder formulation. More preferably, the particles of a tobacco flavoured dry powder formulation in accordance with the present invention preferably comprise less than or equal to about 3 percent by weight of nicotine based on the weight of the tobacco flavoured dry powder formulation.

In preferred embodiments, the particles of a tobacco flavoured dry powder formulation in accordance with the present invention comprise less than or equal to about 3 percent by weight of nicotine based on the weight of the tobacco flavoured dry powder formulation, more preferably less than or equal to about 2.5 percent by weight of nicotine based on the weight of the tobacco flavoured dry powder formulation, even more preferably less than or equal to about 2 percent by weight of nicotine based on the weight of the tobacco flavoured dry powder formulation.

In particularly preferred embodiments, the particles of a tobacco flavoured dry powder formulation in accordance with the present invention comprise less than or equal to about 1.5 percent by weight of nicotine based on the weight of the tobacco flavoured dry powder formulation, more preferably less than or equal to about 1 percent by weight of nicotine based on the weight of the tobacco flavoured dry powder formulation, even more preferably less than or equal to about 0.5 percent by weight of nicotine based on the weight of the tobacco flavoured dry powder formulation.

In some embodiments, the particles of a tobacco flavoured dry powder formulation in accordance with the present invention comprise at least about 0.01 percent by weight of nicotine based on the weight of the tobacco flavoured dry powder formulation or at least about 0.02 percent by weight of nicotine based on the weight of the tobacco flavoured dry powder formulation or at least about 0.05 percent by weight of nicotine based on the weight of the tobacco flavoured dry powder formulation. By way of example, the particles of a tobacco flavoured dry powder formulation in accordance with the present invention comprise at least about 0.06 percent by weight or 0.07 percent by weight or 0.08 percent by weight or 0.09 percent by weight or 0.1 percent by weight of nicotine based on the weight of the tobacco flavoured dry powder formulation.

In some embodiments, the liquid tobacco flavouring composition may be subjected to an additional extraction step - such as, for example, by a liquid-liquid extraction process - for selectively removing nicotine or other alkaloids or both from the liquid tobacco flavouring composition (denicotinisation). This may advantageously enable control of the level of nicotine in the particles of a tobacco flavoured dry powder formulation in accordance with the present invention, such that the particles of a tobacco flavoured dry powder formulation comprise less than about 1 percent by weight of nicotine based on the weight of the tobacco flavoured dry powder formulation. Processes and conditions for achieving denicotinisation of a liquid tobacco extract are known to the skilled person.

In other embodiments, the tobacco starting material may be subjected to a preliminary denicotinisation process. Denicotinisation of tobacco is a well-known process, and has been described in US 200855 A and US 3110315 A.

In further embodiments, the tobacco starting material may be one having a low nicotine content. Examples of low-nicotine tobacco starting material have been described in US 2017/0166913, US 2017/0145432, and AU 2015/202209. Preferably, a ratio by weight of (furaneol + (2,3-diethyl-5-methylpyrazine)*100)) to (nicotine) in the tobacco flavoured dry powder formulation is at least about 5 × 10⁻⁴. More preferably, a ratio by weight of (furaneol + (2,3-diethyl-5-methylpyrazine)*100)) to (nicotine) in the tobacco flavoured dry powder formulation is at least about 8 × 10⁻⁴. Even more preferably, a ratio by weight of (furaneol + (2,3-diethyl-5-methylpyrazine)*100)) to (nicotine) in the tobacco flavoured dry powder formulation is at least about 1 × 10⁻³.

Preferably, a ratio by weight of (furaneol + (2,3-diethyl-5-methylpyrazine)*100)) to (nicotine) in the tobacco flavoured dry powder formulation is less than or equal to about 9 × 10⁻³. More preferably, a ratio by weight of (furaneol + (2,3-diethyl-5-methylpyrazine)*100)) to (nicotine) in the tobacco flavoured dry powder formulation is less than or equal to about 5 × 10⁻³.

In some embodiments, a ratio by weight of (furaneol + (2,3-diethyl-5-methylpyrazine)*100)) to (nicotine) in the tobacco flavoured dry powder formulation is from about 5 × 10⁻⁴ to about 9 × 10⁻³. More preferably, a ratio by weight of (furaneol + (2,3-diethyl-5-methylpyrazine)*100)) to (nicotine) in the tobacco flavoured dry powder formulation is from about 8 × 10⁻⁴ to about 9 × 10⁻³. Even more preferably, a ratio by weight of (furaneol + (2,3-diethyl-5-methylpyrazine)*100)) to (nicotine) in the tobacco flavoured dry powder formulation is from about 1 × 10⁻³ to about 9 × 10⁻³.

In other embodiments, a ratio by weight of (furaneol + (2,3-diethyl-5-methylpyrazine)*100)) to (nicotine) in the tobacco flavoured dry powder formulation is from about 5 × 10⁻⁴ to about 5 × 10⁻³. More preferably, a ratio by weight of (furaneol + (2,3-diethyl-5-methylpyrazine)*100)) to (nicotine) in the tobacco flavoured dry powder formulation is from about 8 × 10⁻⁴ to about 5 × 10⁻³. Even more preferably, a ratio by weight of (furaneol + (2,3-diethyl-5-methylpyrazine)*100)) to (nicotine) in the tobacco flavoured dry powder formulation is from about 1 × 10⁻³ to about 5 × 10⁻³. Particles having a ratio by weight of (furaneol + (2,3-diethyl-5-methylpyrazine)*100)) to (nicotine) in the ranges described above may be obtained by combining a base material with a tobacco flavouring composition wherein a ratio by weight of (furaneol + (2,3-diethyl-5-methylpyrazine)*100)) to (nicotine) falls in the ranges described above.

A dry tobacco flavoured powder formulation as described above may be produced by a method comprising a first step of preparing a tobacco starting material. Preferably, the tobacco starting material is a natural tobacco material.

As will be explained in detail below, by controlling the combination of extraction temperature and time, the composition of the liquid tobacco flavouring composition can be adjusted depending on the desired characteristics of the dry tobacco flavoured powder formulation. In particular, the proportion of specific tobacco compounds within the dry tobacco flavoured powder formulation can be adjusted to a certain degree through the selection of the extraction parameters in order to maximise the ratio of desirable to undesirable tobacco compounds within the liquid tobacco flavouring composition obtained from the extraction steps of the method.

The method comprises a second step of heating the tobacco starting material at an extraction temperature of between 100 degrees Celsius and 160 degrees Celsius for at least 90 minutes. It has been found that below this range insufficient levels of certain flavour compounds are released from the tobacco starting material such that the resultant liquid tobacco extract lacks the desired flavour characteristics. On the other hand, if the tobacco starting material is heated to a temperature above this defined range, unacceptably high levels of certain undesirable tobacco compounds may be released. In general, upon heating the natural tobacco material, any moisture present in the natural tobacco material is also released with the volatile species in the form of steam.

Preferably, the extraction temperature is at least about 110 degrees Celsius, more preferably at least about 115 degrees Celsius, more preferably at least about 120 degrees Celsius, more preferably at least about 125 degrees Celsius.

Preferably, the extraction temperature is less than or equal to about 150 degrees Celsius, more preferably less than or equal to about 145 degrees Celsius, more preferably less than or equal to about 140 degrees Celsius, most preferably less than or equal to about 135 degrees Celsius.

For example, the extraction temperature may be between about 110 degrees Celsius and 150 degrees Celsius, or between about 120 degrees Celsius and about 140 degrees Celsius, or between about 125 degrees Celsius and about 135 degrees Celsius, or about 130 degrees Celsius. An extraction temperature of around 130 degrees Celsius has been found to provide a particularly optimised ratio of desirable to undesirable compounds in the liquid tobacco flavouring composition.

The extraction temperature may be between about 110 degrees Celsius and about 130 degrees Celsius, or between about 115 degrees Celsius and about 125 degrees Celsius, or about 120 degrees Celsius.

The extraction temperature may be between about 125 degrees Celsius and about 155 degrees, more preferably between about 135 degrees Celsius and about 145 degrees Celsius, or about 140 degrees Celsius.

The tobacco starting material is heated at the extraction temperature for at least about 30 minutes or for at least 60 minutes or for at least about 90 minutes, more preferably for at least about 120 minutes. This extraction time is sufficiently long that the desired tobacco flavour compounds can be extracted efficiently to provide a liquid tobacco flavouring composition that can be combined with a base material to produce a dry tobacco flavoured powder formulation having the desired flavour characteristics.

Preferably, the tobacco starting material is heated at the extraction temperature for no more than about 270 minutes, more preferably no more than about 180 minutes.

For example, the tobacco starting material may be heated for between about 90 minutes and about 270 minutes, or between about 120 minutes and about 180 minutes.

The heating time indicated above corresponds to the duration of time over which the tobacco starting material is heated at the extraction temperature, and does not include the time taken to increase the temperature of the tobacco starting material up to the extraction temperature.

The extraction temperature and the duration of heating may be selected within the ranges defined above depending upon factors such as the type of tobacco, possible other components of the tobacco starting material, the desired composition of the liquid tobacco extract. Optionally, the extraction temperature and the duration of heating may be selected within the ranges defined above depending upon a desired level of nicotine in the dry tobacco flavoured powder formulation.

For a specific tobacco compound, the variation in the level of release of the compound with extraction temperature during the extraction process can be readily determined for any given tobacco starting material.

By way of example, it has been found that the level of desirable tobacco flavour compounds, such as β-damascenone and β-ionone, released from a tobacco material will increase with increasing extraction temperature up to a certain peak extraction temperature, after which the level will begin to decrease. The peak extraction temperature for such flavour compounds is typically within the range of 100 degrees Celsius to 160 degrees Celsius such that the level of desirable flavour compounds can be effectively optimised in the extraction method of the present invention.

The level of many undesirable tobacco compounds has been found to increase slowly with increasing extraction temperature up to a threshold temperature, beyond which a rapid increase is observed. This applies, for example, to the level of phenolic compounds, TSNAs and pyrazines and in the case of Bright tobaccos, to the level of furans and formaldehyde. In many cases, the threshold temperature is within the range of 100 degrees Celsius to 160 degrees Celsius and therefore the level of the undesirable compounds can be effectively controlled by adjusting the extraction conditions in the manufacturing method of the present invention.

In some embodiments, the extraction temperature is selected to provide a ratio by weight of (β-ionone + β-damascenone) to (phenol) in the tobacco flavouring composition of at least about 0.25.

Preferably, the extraction temperature or the extraction time or both the extraction temperature and the extraction time are selected to provide a ratio by weight of (β-ionone + β-damascenone) to (phenol) of at least about 0.5, even more preferably at least about 1, most preferably at least about 1.5 in the liquid tobacco flavouring composition. More preferably, the extraction temperature or the extraction time or both the extraction temperature and the extraction time are selected to provide a ratio by weight of (β-ionone + β-damascenone) to (phenol) of at least about 2 and most preferably such that ratio by weight of (β-ionone + β-damascenone) to (phenol) is from about 2 to about 10 or from about 2 to about 5 in the liquid tobacco flavouring composition.

β-damascenone and β-ionone are desirable compounds associated with tobacco flavour. It has been found that the amount of β-damascenone and β-ionone released from a tobacco material will increase with increasing the extraction temperature up to a certain peak extraction temperature, after which the level will begin to decrease. The peak extraction temperature for such flavour compounds is typically within the range of 100 degrees Celsius to 160 degrees Celsius such that the level of desirable flavour compounds in the dry powder formulation can be effectively tailored and controlled during manufacturing.

In some embodiments, the extraction temperature or the extraction time or both the extraction temperature and the extraction time are selected to provide a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition of at least about 1.5. This ratio is higher when the amount of desirable flavourant compounds β-ionone and β-damascenone is higher, or when the amount of TSNAs and 2-furanemethanol is lower.

The ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition may be at least about 0.2, such as at least about 0.5.

In some embodiments, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is at least about 1. Preferably, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is at least about 1.5. More preferably, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is at least about 2. Even more preferably, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is at least about 2.5.

Preferably, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is less than or equal to about 10. More preferably, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is less than or equal to about 6. Even more preferably, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is less than or equal to about 4.

In preferred embodiments, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is from about 1.5 to about 10, more preferably from about 2 to about 10, even more preferably from about 2.5 to about 10. In other embodiments, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is from about 1.5 to about 6, more preferably from about 2 to about 6, even more preferably from about 2.5 to about 6. In further embodiments, the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition is from about 1.5 to about 4, more preferably from about 2 to about 4, even more preferably from about 2.5 to about 4.

Preferably, the extraction temperature or the extraction time or both the extraction temperature and the extraction time are selected to provide a ratio by weight of (furaneol + (2,3-diethyl-5-methylpyrazine)*100)) to (nicotine) of at least about 5 × 10⁻⁴ in the tobacco flavouring composition. More preferably, the extraction temperature or the extraction time or both the extraction temperature and the extraction time are selected to provide a ratio by weight of (furaneol + (2,3-diethyl-5-methylpyrazine)*100)) to (nicotine) of at least about 8 × 10⁻⁴, even more preferably at least about 1 × 10⁻³ in the tobacco flavouring composition. The extraction temperature or the extraction time or both the extraction temperature and the extraction time are preferably selected to provide a ratio by weight of (furaneol + (2,3-diethyl-5-methylpyrazine)*100)) to (nicotine) of less than or equal to about 9 × 10⁻³, more preferably less than or equal to about 5 × 10⁻³ in the tobacco flavouring composition. In some preferred embodiments, the extraction temperature or the extraction time or both the extraction temperature and the extraction time are selected to provide a ratio by weight of (furaneol + (2,3-diethyl-5-methylpyrazine)*100)) to (nicotine) of from about 8 × 10⁻⁴ to about 9 × 10⁻³ or from about 8 × 10⁻⁴ to about 5 × 10⁻³ or from about 1 × 10⁻³ to about 9 × 10⁻³ or from about 1 × 10⁻³ to about 5 × 10⁻³ in the tobacco flavouring composition.

The heating step is preferably carried out in an inert atmosphere. Preferably, a flow of an inert gas, such as nitrogen, is passed through the starting tobacco material during the heating step. As an alternative, the inert gas can be used in combination with water or steam. The volatile tobacco compounds are released into the flow of inert gas or the flow of inert gas and water or steam during the heating step such that the inert gas acts as a carrier for the volatile components.

The flow of inert gas helps convey the steam generated by evaporation of the moisture content of the natural tobacco material and the volatile species - including, in particular, nicotine or flavour-associated compounds or both - out of the extraction equipment.

Further, use of a flow of inert gas, such as nitrogen, under light over-pressure in the extraction equipment has the benefit of preventing the presence of oxygen within the extraction equipment. This is also achievable by heating the natural tobacco material under vacuum. Such benefit is desirable in that it prevents risk of any, even partial, combustion of the natural tobacco material during the heating step. Uncontrolled combustion of the natural tobacco material would clearly be undesirable as it would represent a major safety risk within the manufacturing environment. However, the inventors have found that even a limited, partial combustion of the natural tobacco material may lead to a decrease in the quality of the tobacco extract obtainable by the method, which would be undesirable.

Without wishing to be bound by theory, it is understood that, by preventing combustion of the natural tobacco material, the formation of any undesirable combustion by-products is also prevented. Further, as conditions that would be conducive to combustion of the natural tobacco material are prevented, the natural tobacco material is effectively heated under conditions that mimic, to an extent, conditions under which a tobacco-containing substrate (e.g. homogenised tobacco material) is typically heated in "heat-not-burn" articles. As a result, selective extraction of the flavour-bearing volatile species responsible for the taste consumers associate with heated tobacco is advantageously favoured.

Therefore, by carrying out the heating step in an inert atmosphere the extraction efficiency, product quality and manufacturing safety are advantageously enhanced.

The inert gas flow rate may be optimised based on the scale and geometry of the extraction chamber. A relatively high flow rate of inert gas may advantageously further improve the efficiency of extraction from the tobacco starting material.

The addition of water or steam to the tobacco during extraction has been found to increase yield of extracted components. However, excess addition of water or steam may lead to processing difficulties such as stickiness of the tobacco material.

Optionally, the heating step may be carried out under vacuum.

Suitable heating methods for carrying out the heating of the tobacco starting material would be known to the skilled person and include but are not limited to: dry distillation, hydrodistillation, vacuum distillation, flash distillation and thin film hydrodistillation.

The liquid tobacco flavouring composition may be prepared from a tobacco starting material consisting of a single type of natural tobacco. Alternatively, the tobacco starting material may comprise a blend of two or more types of natural tobaccos. The ratio of the different tobacco types may be adapted depending on the desired flavour characteristics of the tobacco flavoured dry powder formulation to be manufactured from the liquid tobacco flavouring composition. For example, where it is desired to provide a relatively high level of nicotine, the proportion of Burley tobacco may be increased.

Where it is desired to produce a liquid tobacco flavouring composition from a combination of two of more different tobacco types, the tobacco types may be heated separately at different extraction temperatures within the defined range of 100 degrees Celsius to 160 degrees Celsius, or a mixture of the tobacco types may be heated together at a single extraction temperature within the range.

The tobacco starting material may be a solid tobacco material, such as a powder, leaf scraps or shreds, or intact leaf. Alternatively, the tobacco starting material may be a liquid tobacco material such as a dough, gel, slurry, or suspension.

The tobacco starting material may be derived from any suitable tobacco material, including but not limited to tobacco leaf, tobacco stem, reconstituted tobacco, cast tobacco, extruded tobacco or tobacco derived pellets.

Preferably, in the step of preparing the tobacco starting material, the tobacco is ground or cut in order to reduce the size of tobacco particles within the tobacco starting material. This may advantageously improve the homogeneity of heating of the tobacco starting material and the efficiency of the extraction.

The tobacco starting material may optionally be dried prior to the heating step in order to decrease the water content of the tobacco starting material. Drying of the tobacco starting material may be carried out by any suitable chemical or physical drying process. Alternatively, water may be added to the tobacco starting material prior to the heating step in order to increase the water content of the tobacco starting material.

In certain embodiments of the present invention, the step of preparing the tobacco starting material may comprise the step of impregnating the tobacco starting material with an aerosol former. When this impregnation of the tobacco starting material is carried out prior to the heating step, it may advantageously increase the amount of certain desirable tobacco compounds that are released from the tobacco starting material upon heating. For example, impregnation of the tobacco starting material with glycerin has been found to advantageously increase the amount of nicotine that is extracted from the tobacco starting material. In another example, impregnation of the tobacco starting material with a polar aerosol former such as a mixture of polyethylene glycol and vegetal glycerin, or triacetin, has been found to advantageously increase the amount of flavour compounds that are extracted from the tobacco starting material.

Optionally, the tobacco starting material may be digested enzymatically prior to the heating step. This has been found to provide a significant increase in the yield of certain flavour compounds from the tobacco starting material.

The tobacco starting material may optionally be analysed prior to the heating step in order to determine the composition, for example, the content of reducing sugars of alkaloids. This information about the composition may helpfully be used to select an appropriate extraction temperature.

Preferably, in the step of preparing the natural tobacco material, the tobacco is not subjected to any treatment adapted to alter the pH of the tobacco. In particular, in the step of preparing the natural tobacco material, the tobacco is not subjected to any treatment adapted to significantly increase the pH of the tobacco. For example, the natural tobacco material is not contacted with an aqueous solution containing a salt of an alkali or alkali-earth metal. Advantageously it has been found that maintaining the tobacco material in a less modified state may provide a more authentic or more natural flavour profile which may be appreciated by a consumer. Further, the inventors have found that subjecting the natural tobacco material to a treatment adapted to increase the pH of the tobacco, such as an alkali treatment, prior to heating the tobacco material as part of the extraction process leads to lower levels of desirable heated tobacco flavour compounds in the liquid tobacco extract. By way of example, not subjecting the natural tobacco material to an alkali treatment has been found to cause be associated with a significant increase in the weight ratio of (β-ionone + β-damascenone) to (phenol) in the liquid tobacco extract compared with an equivalent, alkali-treated natural tobacco material.

During the heating of the tobacco starting material, the volatile compounds released from the tobacco starting material are collected using any suitable technique. Where the tobacco starting material is heated in a flow of an inert gas, as described above, the volatile compounds are collected from the inert gas flow. Different collection methods would be well known to the skilled person. In view of the collecting step, heating the natural tobacco material in a flow of inert gas or a flow comprising an inert gas and water or stream has the additional benefit that the inert gas flow containing the volatile compounds may be more easily directed into a container containing an extraction solvent, such as a non-aqueous extraction liquid solvent.

Preferably, the step of collecting the volatile compounds is carried out using a condensation technique in which the volatile compounds are condensed and the condensate is collected.

In some embodiments, the condensate obtained is added to a liquid aerosol former, preferably propylene glycol (PG).

The addition of a liquid aerosol former, and particularly addition of PG, may advantageously prevent the condensed volatile compounds from splitting into two phases or forming an emulsion, as some tobacco constituents would tend to do. Without wishing to be bound by theory, the inventors have observed that the solubility of the tobacco constituents in the hydrolate (i.e. the aqueous fraction of the liquid, naturally derived tobacco extract) depends primarily on their polarity, on their concentration and on the pH of the hydrolate, which may vary depending on the tobacco type. As a result, an oily layer tends to form at the surface of the liquid tobacco flavouring composition, if the amount of aerosol former is not sufficient. Such oily material can aggregate at different locations on the equipment used to carry out the extraction process. The addition of a liquid aerosol former, such as PG, helps prevent the formation of such layer and favours homogenisation of the liquid tobacco flavouring composition.

In addition, the liquid aerosol former advantageously helps trap the flavour-associated compounds independent of their polarity and volatility. Use of PG as the aerosol former for the condensation and collection step has the further advantage that, by reducing the water activity of aqueous solutions, PG exerts an anti-microbial activity. By adjusting the content of PG in the liquid tobacco flavouring composition, it is therefore also possible to ensure that the composition substantially does not undergo any microbial activity.

A condensation technique is one that removes volatile compounds from a gaseous stream by saturating the volatile compounds in the gaseous stream. Condensation, refrigeration, and cryogenic systems are usually used on gaseous streams that contain only volatile organic compounds. Saturation (dew point temperature) occurs when the partial pressure of the volatile compound is equal to its vapour pressure. Once saturation has been attained, separation via condensation occurs by either increasing the system pressure at constant temperature (known as compression condensation) or by lowering the temperature at constant temperature (known as refrigerated condensation).

Preferably, in methods in accordance with the present invention, the step of collecting the volatile compounds is carried out using a refrigerated condensation technique. This may be attained either by direct contact between the gaseous stream containing the volatile compounds and a cooling liquid. Alternatively, this may be attained by indirect contact via a heat exchanger between the gaseous stream containing the volatile compounds and a cooling medium. For direct contact applications, a cryogenic gas such as liquid nitrogen may be injected into the gaseous stream. Indirect cooling condensation may be preferred as direct cooling condensation may require an additional separation stage.

By way of example, in methods in accordance with the present invention, condensation of the volatile compounds may be carried out using any suitable apparatus, for example, in a refrigerated column.

However, as the extraction process is typically carried out at temperatures from about 130 degrees Celsius to about 160 degrees Celsius, a bland cooling of the gaseous stream with air at room temperature is generally sufficient to cause condensation of the extracted volatile compounds.

In an alternative embodiment, the step of collecting the volatile compounds may use an absorption technique in which the volatile compounds are trapped in a liquid solvent. For example, an inert gas flow containing the volatile compounds may be directed into a container of a liquid solvent. The liquid solvent may be an aerosol former such as triacetin, glycerin, polyethylene glycol or combinations thereof. Preferably, the liquid solvent is retained at a temperature of less than 0 degrees Celsius in order to optimise the transfer of the volatile compounds into the liquid solvent.

As a further alternative, the step of collecting the volatile compounds may be carried out using an adsorption technique in which the volatile compounds are adsorbed onto the surface of a solid adsorbent material, such as activated carbon. The adsorbed compounds may then be transferred into a liquid solvent.

In the method of the present invention, the next step is the formation of a liquid tobacco flavouring composition from the collected volatile compounds. The nature of this step may depend upon the collection method. The "collected volatile compounds" may be in the form of a solution of the tobacco derived volatile compounds in a liquid solvent or carrier.

Where the volatile compounds are collected by condensation, the step of forming the liquid tobacco flavouring composition may comprise adding the condensate to a liquid solvent, such as an aerosol former.

Alternatively, where the volatile compounds are collected by absorption in a liquid solvent, as described above, the step of forming the liquid tobacco flavouring composition preferably comprises drying the solution of the volatile compounds in the liquid solvent in order to concentrate the solution. This may be carried out, for example, in order to arrive at a desired concentration of flavour compounds. Drying may be carried out using any suitable means, including but not limited to desiccation, molecular sieves, freeze drying, phase separation, distillation, membrane permeation, controlled crystallisation of water and filtering, reverse hygroscopicity, ultracentrifugation, liquid chromatography, reverse osmosis or chemical drying.

In preferred embodiments, the solution of the volatile compounds in a liquid solvent is concentrated by desiccation.

Optionally, the step of forming the liquid tobacco flavouring composition comprises a filtering step.

Optionally, the step of forming the liquid tobacco flavouring composition comprises a blending step in which extracts derived from different tobacco starting materials are combined.

Optionally, the step of forming the liquid tobacco flavouring composition comprises adding one or more additives, such as an organic acid, to the solution of volatile compounds. However, in many cases the liquid tobacco flavouring composition is suitable for use without the inclusion of additives.

In methods in accordance with the present invention, the tobacco flavouring composition is combined with a base material to form the plurality of particles of the dry tobacco flavoured formulation.

The base material may comprise one or more of a gum, such as Arabic gum, guar seed meal, locust bean meal, khataya, ghatti, tragacanth, xanthan; a starch, a hydrolysed starch such as maltodextrins and corn syrup solids or glucose syrups, a chemically modified starch, carboxymethyl cellulose, a sugar, such as a monosaccharide, a disaccharide or a polysaccharide. Examples of suitable sugars include, but are not limited to, lactose, sucrose, raffinose, trehalose, fructose, dextrose, glucose, maltose, mannitol, or combinations thereof. Particularly preferred sugars include trehalose or mannitol.

The mono and disaccharides such as sucrose, lactose, and glucose or sugar alcohols such as sorbitol can be used in blends with chemically modified starch or gum Arabic to impart improved stability against tobacco extract oxidation. Hydrolysed starches have marginal retention of lipophilic volatiles but are very well suited as carriers for hydrophilic volatiles. Emulsifying starches have better lipophilic properties and provide emulsification properties and excellent volatile retention during spray drying, but poor protection of the tobacco extract flavouring to oxidation. Arabic gum is an excellent encapsulating material, a very good emulsifier and provides good retention of volatiles during the drying process of the manufactured tobacco flavored powders. Particularly preferred are base materials with good encapsulation properties such as maltodextrin and cyclomaltodextrin.

In an embodiment, the step of combining the base material and the tobacco flavouring composition to form tobacco flavoured particles comprises a first step of forming a mixture of the base material and the liquid tobacco flavouring composition; a second step of freezing the mixture; a third step of drying the frozen mixture; and a fourth step of grinding the dried mixture to form the tobacco flavoured particles.

In another embodiment, the step of combining the base material and the tobacco flavouring composition to form tobacco flavoured particles comprises forming a mixture of the base material and the liquid tobacco flavouring composition and spray-drying the mixture to form the tobacco flavoured particles.

Particles of a dry tobacco flavoured powder formulation as described above may find use in a powder system comprising a further plurality of particles. In some embodiments, a powder system in accordance with the present invention may comprise a first plurality of particles as described above or obtained by a method as described above or both, and having a particle size of at least about 20 micrometres in combination with a second plurality of particles comprising nicotine and having a particle size of about 10 micrometres or less.

Without wishing to be bound by theory, it is understood that the larger, tobacco flavour-bearing particles, are adapted for deposition in the consumer's mouth, whereas the smaller, nicotine-containing particles are adapted to reach the consumer's lungs when inhaled.

In preferred embodiments, the particles of the second plurality of particles comprise nicotine, a sugar or an amino acid or both. Preferably, the particles of the second plurality of particles comprise nicotine, a sugar and an amino acid. The term "amino acid" is used herein with reference to the present invention to describe a single unmodified or modified amino acid moiety, preferably unmodified.

In another embodiment, a powder system in accordance with the present invention comprises a first plurality of tobacco flavoured particles having a particle size of at least about 20 micrometres and a second plurality of particles having a particle size of less than about 20 micrometres, wherein a first ratio by weight of (β-ionone + β-damascenone) to (phenol) in the tobacco flavoured particles of the first plurality is greater than 0.25.

Preferably, the ratio by weight of (β-ionone + β-damascenone) to (phenol) in the tobacco flavoured particles of the first plurality is greater than 0.5.

Preferably, a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavoured particles of the first plurality is greater than 1.5.

Further, the tobacco flavoured particles of the first plurality may comprise one or more of furaneol, 2,3-diethyl-5-methylpyrazine, acetic acid, vanillin, 2-ethyl-3,5-dimethylpyrazine, 2-methylbutanoic acid, 3-methylbutanoic acid, 3-methyl-2,4-nonanedione, 2-methoxyphenol, 2-phenylethanol, eugenol and sotolone.

A powder system in accordance with the present invention may comprise at least about 5 percent by weight of the first particles. Preferably, the powder system comprises at least about 10 percent by weight of the first particles. More preferably, the powder system comprises at least about 15 percent by weight of the first particles.

The powder system may comprise less than or equal to about 50 percent by weight of the first particles. Preferably, the powder system comprises less than or equal to about 45 percent by weight of the first particles. More preferably, the powder system comprises less than or equal to about 25 percent by weight of the first particles.

In some embodiments, the powder system comprises from about 5 percent by weight to about 50 percent by weight of the first particles, more preferably from about 10 percent by weight to about 50 percent by weight and even more preferably from about 15 percent by weight to about 50 percent by weight of the first particles. In other embodiments, the powder system comprises from about 5 percent by weight to about 45 percent by weight of the first particles, more preferably from about 10 percent by weight to about 45 percent by weight and even more preferably from about 15 percent by weight to about 45 percent by weight of the first particles. In further embodiments, the powder system comprises from about 5 percent by weight to about 25 percent by weight of the first particles, more preferably from about 10 percent by weight to about 25 percent by weight and even more preferably from about 15 percent by weight to about 25 percent by weight of the first particles.

The powder system may comprise at least about 50 percent by weight of the second particles. Preferably, the powder system comprises at least about 65 percent by weight of the second particles. More preferably, the powder system comprises at least about 75 percent by weight of the second particles.

The powder system may comprise less than or equal to about 95 percent by weight of the second particles. Preferably, the powder system comprises less than or equal to about 90 percent by weight of the second particles. More preferably, the powder system comprises less than or equal to about 85 percent by weight of the second particles.

In some embodiments, the powder system comprises from about 50 percent by weight to about 95 percent by weight of the second particles, more preferably from about 65 percent by weight to about 95 percent by weight and even more preferably from about 75 percent by weight to about 95 percent by weight of the second particles. In other embodiments, the powder system comprises from about 50 percent by weight to about 90 percent by weight of the second particles, more preferably from about 65 percent by weight to about 90 percent by weight and even more preferably from about 75 percent by weight to about 90 percent by weight of the second particles. In further embodiments, the powder system comprises from about 50 percent by weight to about 85 percent by weight of the second particles, more preferably from about 65 percent by weight to about 85 percent by weight and even more preferably from about 75 percent by weight to about 85 percent by weight of the second particles.

In a powder system in accordance with the present invention a weight ratio of the second plurality of particles to the first plurality of particles may be at least about 1:1, preferably at least 2:1, more preferably about 3:1.

In a powder system in accordance with the present invention a weight ratio of the second plurality of particles to the first plurality of particles may be less than or equal to about 10:1, preferably less than or equal to 8:1, more preferably less than or equal to 6:1, even more preferably less than or equal to 5:1.

In some embodiments, a weight ratio of the second plurality of particles to the first plurality of particles is preferably from about 1:1 to about 8:1, more preferably from about 2:1 to about 8:1, even more preferably from about 3:1 to about 8:1. In other embodiments, a weight ratio of the second plurality of particles to the first plurality of particles is preferably from about 1:1 to about 6:1, more preferably from about 2:1 to about 6:1, even more preferably from about 3:1 to about 6:1. In further embodiments, a weight ratio of the second plurality of particles to the first plurality of particles is preferably from about 1:1 to about 5:1, more preferably from about 2:1 to about 5:1, even more preferably from about 3:1 to about 5:1. By way of example, a weight ratio of the second plurality of particles to the first plurality of particles may be about 4:1.

Preferably, the first plurality of particles and the second plurality of particles form at least about 90 percent by weight, or at least about 95 percent by weight, or at least about 99 percent by weight, or 100 percent by weight of the total weight of the powder system.

The first plurality of particles may have a particle size of at least about 20 micrometres, preferably at least about 50 micrometres, more preferably at least about 75 micrometres, even more preferably at least about 100 micrometres. The first plurality of particles preferably have a particle size of less than or equal to about 200 micrometres. More preferably, the first plurality of particles have a particle size of less than or equal to about 150 micrometres.

The first plurality of particles preferably have a particle size from about 20 micrometres to about 200 micrometres, more preferably from about 50 micrometres to about 200 micrometres, even more preferably from about 75 micrometres to about 200 micrometres. In other embodiments, the first plurality of particles have a particle size from about 20 micrometres to about 150 micrometres, more preferably from about 50 micrometres to about 150 micrometres, even more preferably from about 75 micrometres to about 150 micrometres.

The second plurality of particles may have a particle size of less than or equal to about 10 micrometres, preferably less than or equal to about 5 micrometres, more preferably less than or equal to about 3 micrometres.

As described briefly above, the particles of the second plurality of particles preferably comprise nicotine, a sugar and an amino acid. The amino acid may reduce adhesion forces of the particles and mitigate or prevent agglomeration of the particles during formation or subsequent handling. The second plurality of particles may be a free-flowing material and may have a stable relative particle size distribution during processing, transport and storage.

Useful amino acids may include leucine, alanine, valine, isoleucine, methionine, phenylalanine, tyrosine, tryptophan, or a combination thereof. One preferred amino acid is leucine or a leucine isomer, such as L-leucine. An example of a preferred peptide is trileucine.

The particle may include a sugar. Sugar refers to simple sugars, monosaccharides, disaccharides, and polysaccharides. Without limitation, examples of suitable sugars are lactose, sucrose, raffinose, trehalose, fructose, dextrose, glucose, maltose, mannitol, or combinations thereof. Preferred sugars include trehalose or mannitol.

The second plurality of particles may contain less than or equal to about 30 percent by weight nicotine. Preferably, the second plurality of particles contain less than or equal to about 10 percent by weight nicotine. More preferably, the second plurality of particles contain less than or equal to about 7 percent by weight nicotine. Even more preferably, the second plurality of particles contain less than or equal to about 6 percent by weight nicotine.

The second plurality of particles preferably contain at least about 1 percent by weight nicotine. More preferably, the second plurality of particles contain at least about 2 percent by weight nicotine. Even more preferably, the second plurality of particles contain at least about 3 percent by weight nicotine. Most preferably, the second plurality of particles contain at least about 4 percent by weight nicotine.

In some embodiments, the second plurality of particles comprise from about 1 percent by weight nicotine to about 10 percent by weight nicotine, preferably from about 2 percent by weight nicotine to about 10 percent by weight nicotine, more preferably from about 3 percent by weight nicotine to about 10 percent by weight nicotine, even more preferably from about 4 percent by weight nicotine to about 10 percent by weight nicotine.

In other embodiments, the second plurality of particles comprise from about 1 percent by weight nicotine to about 30 percent by weight nicotine, preferably from about 2 percent by weight nicotine to about 25 percent by weight nicotine, more preferably from about 3 percent by weight nicotine to about 20 percent by weight nicotine, even more preferably from about 4 percent by weight nicotine to about 15 percent by weight nicotine.

In other embodiments, the second plurality of particles comprise from about 1 percent by weight nicotine to about 7 percent by weight nicotine, preferably from about 2 percent by weight nicotine to about 7 percent by weight nicotine, more preferably from about 3 percent by weight nicotine to about 7 percent by weight nicotine, even more preferably from about 4 percent by weight nicotine to about 7 percent by weight nicotine.

In further embodiments, the second plurality of particles comprise from about 1 percent by weight nicotine to about 6 percent by weight nicotine, preferably from about 2 percent by weight nicotine to about 6 percent by weight nicotine, more preferably from about 3 percent by weight nicotine to about 6 percent by weight nicotine, even more preferably from about 4 percent by weight nicotine to about 6 percent by weight nicotine.

Nicotine in the nicotine particles may be a pharmaceutically acceptable free-base nicotine, or nicotine salt or nicotine salt hydrate. Useful nicotine salts or nicotine salt hydrates include nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. The compound combining with nicotine to form the salt or salt hydrate may be chosen based on its expected pharmacological effect.

The nicotine content is calculated based on the total amount of nicotine regardless of the form of nicotine. For example, the second plurality of particles may include 8.4 percent by weight of a nicotine salt such as nicotine lactate, but the nicotine content in the second plurality of particles is thus 5 percent by weight.

Methods are available to the skilled person to assess whether a powder system is a powder system in accordance with the present invention. One such method comprises a first step of assessing whether the powder system comprises a first plurality of particles having a particles size of at least about 20 micrometres in combination with a second plurality of particles having a particle size of less than about 20 micrometres, such as a particle size of about 10 micrometres or less. By way of example, one such first step may involve using laser diffraction or laser scattering in order to ascertain firstly whether the powder system has a size distribution that is bimodal or polymodal, and whether a population of particles is present that have a particle size of 20 micrometres or more.

Further, one such method comprises a second step of separating the plurality of particles having a size of at least about 20 micrometres from the smaller particles. One such second step may for example involve using an impactor or sieves in order to separate the particles based on their size, such that the particles having a particle size of at least about 20 micrometres can be grouped together.

In addition, one such method comprises a third step of analysing the particles having a particle size of at least about 20 micrometres to ascertain whether a ratio by weight of (β-ionone + β-damascenone) to (phenol) in the particles of the first plurality is greater than 0.25.

An embodiment of the present invention will now be further described, by way of example only.

### Example 1

A tobacco starting material is prepared from a flue-cured Bright tobacco material. The tobacco material is cut to form tobacco shreds having dimensions of 2.5 millimetres by 2.5 millimetres and the tobacco shreds are loaded into an extraction chamber, without compression. The tobacco starting material is heated within the extraction chamber to a temperature of 130 degrees Celsius for a period of 3 hours. During heating, a flow of nitrogen is passed through the extraction chamber at a flow rate of about 40 litres per minute.

The volatile compounds released from the tobacco starting material during the heating step are collected by absorption into a liquid solvent formed of propylene glycol at minus 10 degrees Celsius and with agitation of 750 rpm.

Thus a liquid tobacco flavouring composition is obtained directly from an extraction process at a temperature of 130 degrees Celsius for a period of 3 hours. The liquid tobacco flavouring composition provides an optimised level of desirable flavour compounds such as β - damascenone and β -ionone to undesirable compounds such as phenol, 4-(methylnitrosamino)-1-(3-pyridyl)-1-butanone, (R,S)-N-nitrosoanatabine, (R,S)-N-nitrosoanabasine, N-nitrosonornicotine and 2-furanemethanol. The liquid tobacco flavouring composition further provides a level of desirable flavour compounds such as furaneol and 2,3-diethyl-5-methylpyrazine to nicotine.

The solution of propylene glycol with the collected volatile compounds is concentrated in a desiccation process to reduce the moisture level of the liquid tobacco extract to approximately 15 percent.

### Example 2

This example provides two liquid tobacco flavouring compositions, both of which are obtained directly from an extraction process at a temperature of 130 degrees Celsius for a period of 3 hours.

### Example 2a

Example 2a relates to a liquid tobacco flavouring composition derived from flue-cured Bright tobacco material. The content of the concentrated liquid tobacco flavouring composition of Example 2a is as follows:
▪ Nicotine: 0.53 % w/w
▪ Propylene Glycol: 91.8 % w/w
▪ Water: 6.3 % w/w
▪ Balance (including flavourants as detailed in Table 1 below): 1.57 % w/w

### Example 2b

Example 2b relates to a liquid tobacco flavouring composition derived from Burley tobacco material. The content of the concentrated liquid tobacco flavouring composition of Example 2b is as follows:
▪ Nicotine: 1.82 % w/w
▪ Propylene Glycol: 89.6 % w/w
▪ Water: 5.7 % w/w
▪ Balance (including flavourants as detailed in Table 1 below): 2.88 % w/w

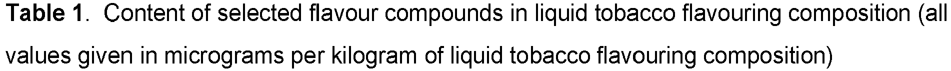

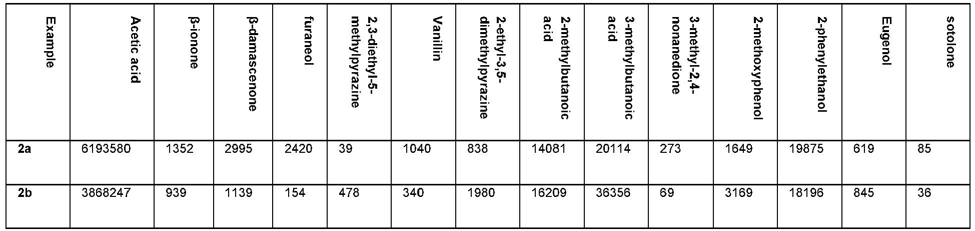

The liquid tobacco flavour compositions of Examples 2a and 2b in accordance with the invention contain acceptably low levels of undesirable compounds such as phenol, 4-(methylnitrosamino)-1-(3-pyridyl)-1-butanone, (R,S)-N-nitrosoanatabine, (R,S)-N-nitrosoanabasine, N-nitrosonornicotine and 2-furanemethanol.

### Example 3

This example provides three liquid tobacco flavouring compositions in accordance with the invention, each of which is a liquid tobacco flavouring composition obtained directly from an extraction process at a temperature of 130 degrees Celsius for a period of 3 hours.

### Example 3a

Example 3a relates to a liquid tobacco flavouring composition derived from oriental Bright tobacco material. The content of the liquid tobacco flavouring composition of Example 3a is as follows:
▪ Nicotine: 0.4 % w/w
▪ Propylene glycol: 84 % w/w
▪ Acetic Acid: 1.0% w/w
▪ Water: 12.5 % w/w
▪ Balance (including flavourants): 2.1 % w/w

### Example 3b

Example 3b relates to a liquid tobacco flavouring composition derived from flue-cured Bright tobacco material. The content of the liquid tobacco flavouring composition of Example 3b is as follows:
▪ Nicotine: 1.2 % w/w
▪ Propylene Glycol: 84 % w/w
▪ Acetic acid: 1.0 % w/w
▪ Water: 12.5 % w/w
▪ Balance (including flavourants): 1.3 % w/w

### Example 3c

Example 3c relates to a liquid tobacco flavouring composition derived from Burley tobacco material. The content of the liquid tobacco flavouring composition of Example 3c is as follows:
▪ Nicotine: 2.6 % w/w
▪ Propylene Glycol: 84 % w/w
▪ Acetic acid: 0.5 % w/w
▪ Water: 12.5 % w/w
▪ Balance (including flavourants): 0.4 % w/w

The liquid tobacco flavouring compositions of Example 3 provide an optimised level of desirable flavour compounds such as β-damascenone and β-ionone to undesirable compounds such as phenol, 4-(methylnitrosamino)-1-(3-pyridyl)-1-butanone, (R,S)-N-nitrosoanatabine, (R,S)-N-nitrosoanabasine, N-nitrosonornicotine and 2-furanemethanol. The liquid tobacco flavouring compositions further provide a level of desirable flavour compounds such as furaneol and 2,3-diethyl-5-methylpyrazine to nicotine.

### Example 4

The liquid tobacco flavouring composition of Example 1 was concentrated in a desiccation process to reduce the moisture level of the liquid tobacco extract to approximately 15 percent.

Glycerine was added to the resultant concentrated liquid tobacco extract, such that the liquid tobacco flavouring composition ultimately contained 20 percent by weight glycerine and 80 percent by weight liquid tobacco extract, based on the weight of the liquid tobacco flavouring composition.

### Example 5

The liquid tobacco flavouring compositions of Examples 1 to 4 are combined with a base material consisting of maltodextrin. A weight ratio of liquid tobacco flavour composition to base material is 30:70.
In more detail, 3 grams of each one of the liquid tobacco flavouring compositions of Examples 1 to 4 are weighed into respective beakers, after 7 grams of maltodextrin are weighed into each one of the beakers.

The two ingredients are stirred to obtain a homogeneous dough-like mixture. The dough-like mixture is spread on a Petri dish, covered with aluminium foil and stored in a freezer for at least 2 hours. Subsequently, the frozen dough-like mixture is introduced into a lyophilisation chamber (freeze-dryer) in order to dehydrate the dough-like mixture. This is done in a two-step process. In a first step of primary drying the dough-like mixture is dried for about 12 hours to allow the ice to sublimate. In a second set of secondary drying the dough-like mixture is dried for a further 2 hours to allow the unfrozen water molecules to be removed.

Prior to start of the drying process the aluminium foil on top of the Petri dish is perforated in order to favour the elimination of water from the dough-like mixture.

The dehydrated dough-like mixture is subsequently transferred into an alumina mortar and ground to form tobacco flavoured particles. A tobacco flavoured dry powder formulation is thus obtained that has a particle size distribution mean of about 50 micrometres to about 60 micrometres.

### Example 6

Examples 6 provides particles of a tobacco flavoured dry powder formulation similar to the particles of Example 5. In contrast to the particles of Example 5, a weight ratio of liquid tobacco flavour composition to base material in the particles of Example 6 is 50:50 (Example 6a) and 20:80 (Example 6b).

### Example 7

Example 7 provides particles of a tobacco flavoured dry powder formulation similar to the particles of Example 5. In contrast to the particles of Example 5 the extract of Example 7 was produced by condensation without the addition of propylene glycol or other solvent. Accordingly, the concentration of the flavour compounds within liquid tobacco flavour composition is significantly high and in light of this a weight ratio of liquid tobacco flavour composition to base material in the particles of Example 7 is 10:90 (Example 7a) and 15:85 (Example 7b).

### Example 8

Three tobacco starting materials are prepared from a flue-cured Bright tobacco material (2A), a Burley tobacco material (2B), and an Oriental tobacco material (2C), respectively.
Each one of the three tobacco materials is cut to form tobacco shreds having dimensions of 2.5 millimetres by 2.5 millimetres, and the tobacco shreds are loaded into an extraction chamber, without compression.

Each one of the tobacco starting materials is heated within the extraction chamber to a temperature of 130 degrees Celsius for a period of 120 minutes. During heating, a flow of nitrogen is passed through the extraction chamber at a flow rate of 2 litres per minute.

The volatile compounds released from each tobacco starting material during the heating step are collected by absorption into a liquid solvent formed of polypropylene glycol at 0 degrees Celsius.

A liquid tobacco extract is obtained directly from such extraction process. Each liquid extract obtained from each one of the three tobacco starting materials is then concentrated under vacuum (50 mbar) at 55 degrees Celsius until a moisture content of 12 percent ± 2 percent is reached.

**Table 2. Value of selected ratios by weight of desirable to undesirable tobacco compounds within the liquid tobacco extracts**

| **Example** | **(β-ionone + β-damascenone) to (phenol)** | **(furaneol + (2,3-diethyl-5-methylpyrazine)*100)) to (nicotine)** | **(β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600))** |
|---|---|---|---|
| **2A** | 2.27 | 1.35 x 10⁻³ | 5.25 |
| **2B** | 2.96 | 1.71 x 10⁻³ | 3.50 |
| **2C** | 4.12 | 2.75 x 10⁻³ | 7.83 |

In all three liquid extracts in accordance with the invention 2A, 2B, and 2C the ratio by weight of (β-ionone + β-damascenone) to (phenol) is consistently and significantly above 2.0. Further, in all three liquid extracts in accordance with the invention 2A, 2B, and 2C the ratio by weight of (furaneol + (2,3-diethyl-5-methylpyrazine)*100)) to (nicotine) is consistently and significantly above 1 × 10⁻³. Additionally, in all three liquid extracts in accordance with the invention 2A, 2B, and 2C the ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) is consistently and significantly above 3.

## Claims

1. A tobacco flavoured dry powder formulation comprising a plurality of particles comprising a base material and a tobacco flavouring composition, wherein a first ratio by weight of (β-ionone + β-damascenone) to (phenol) in the tobacco flavoured dry powder formulation is greater than 0.25.

2. A tobacco flavoured powder formulation according to claim 1 wherein the ratio by weight of (β-ionone + β-damascenone) to (phenol) in the tobacco flavoured dry powder formulation is greater than 0.5.

3. A tobacco flavoured powder formulation according to claim 1 or 2 wherein a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) is greater than 1.5.

4. A tobacco flavoured powder formulation according to any one of the preceding claims further comprising one or more of furaneol, 2,3-diethyl-5-methylpyrazine, acetic acid, vanillin, 2-ethyl-3,5-dimethylpyrazine, 2-methylbutanoic acid, 3-methylbutanoic acid, 3-methyl-2,4-nonanedione, 2-methoxyphenol, 2-phenylethanol, eugenol and sotolone.

5. A method of producing a tobacco flavoured powder formulation, the method comprising the steps of:
preparing a tobacco starting material;
heating the tobacco starting material at an extraction temperature of between 100 degrees Celsius and 160 degrees Celsius for at least 90 minutes;
collecting the volatile compounds released from the tobacco starting material during the heating step;
forming a liquid tobacco flavouring composition comprising the collected volatile compounds;
combining a base material and the liquid tobacco flavouring composition to form tobacco flavoured particles,
wherein in the step of preparing the tobacco starting material, the tobacco starting material is not subjected to any treatment adapted to alter the pH of the tobacco.

6. A method according to claim 5, wherein the tobacco starting material is heated at an extraction temperature of between 120 degrees Celsius and 140 degrees Celsius.

7. A method according to claim 5 or 6, wherein the tobacco starting material is heated at the extraction temperature for at least 120 minutes.

8. A method according to any one of claims 5 to 7, wherein the extraction temperature is selected to provide a ratio by weight of (β-ionone + β-damascenone) to (phenol) in the tobacco flavouring composition of at least about 0.25.

9. A method according to any one of claims 5 to 8, wherein the extraction temperature is selected to provide a ratio by weight of (β-ionone + β-damascenone) to (4-(methylnitrosamino)-1-(3-pyridyl)-1-butanone + (R,S)-N-nitrosoanatabine + (R,S)-N-nitrosoanabasine + N-nitrosonornicotine + ((2-furanemethanol)/600)) in the tobacco flavouring composition of at least about 1.5.

10. A method according to any one of claims 5 to 9, wherein the extraction temperature is selected to provide a ratio by weight of (furaneol + (2,3-diethyl-5-methylpyrazine)*100)) to (nicotine) in the tobacco flavouring composition of at least about 5 × 10⁻⁴.

11. A method according to any one of claims 5 to 10, wherein the base material comprises one or more of a gum, a starch, a hydrolysed starch, a chemically modified starch, carboxymethyl cellulose, a monosaccharide, a disaccharide.

12. A method according to any one of claims 5 to 11, wherein the step of collecting the volatile compounds released from the tobacco starting material during the heating step comprises causing the volatile compounds to condensate by refrigeration.

13. A method according to any one of claims 5 to 12 wherein the step of combining the base material and the liquid tobacco flavouring composition to form tobacco flavoured particles comprises:
forming a mixture of the base material and the liquid tobacco flavouring composition;
freezing the mixture;
drying the frozen mixture; and
grinding the dried mixture to form the tobacco flavoured particles;
or wherein the step of combining the base material and the liquid tobacco flavouring composition to form tobacco flavoured particles comprises:
forming a mixture of the base material and the liquid tobacco flavouring composition;
spray-drying the mixture to form the tobacco flavoured particles.

14. A powder system comprising:
a first plurality of particles according to any one of claims 1 to 4 and having a particle size of at least about 20 micrometres; and
a second plurality of particles having a particle size of about 10 micrometres or less and comprising nicotine.

15. A powder system comprising:
a first plurality of tobacco flavoured particles having a particle size of at least about 20 micrometres and a second plurality of particles having a particle size of less than about 20 micrometres, wherein a first ratio by weight of (β-ionone + β-damascenone) to (phenol) in the tobacco flavoured of the first plurality is greater than 0.25.

## Patentansprüche

1. Trockenpulverformulierung mit Tabakaroma, umfassend eine Vielzahl von Partikeln, die ein Basismaterial und eine Tabakaromazusammensetzung umfassen, wobei ein erstes Gewichtsverhältnis von (β-Ionon + β-Damascenon) zu (Phenol) in der Trockenpulverformulierung mit Tabakaroma größer als 0,25 ist.

2. Pulverformulierung mit Tabakaroma nach Anspruch 1, wobei das Gewichtsverhältnis von (β-Ionon + β-Damascenon) zu (Phenol) in der Trockenpulverformulierung mit Tabakaroma größer als 0,5 ist.

3. Pulverformulierung mit Tabakaroma nach Anspruch 1 oder 2, wobei ein Gewichtsverhältnis von (β-Ionon + β-Damascenon) zu (4-(Methylnitrosamino)-1-(3-pyridyl)-1-butanon + (R,S)-N-Nitrosoanatabin + (R,S)-N-Nitrosoanabasin + N-Nitrosonornicotin + ((2-Furanemethanol)/600)) größer als 1,5 ist.

4. Pulverformulierung mit Tabakaroma nach einem beliebigen der vorhergehenden Ansprüche, ferner umfassend eines oder mehrere von Furaneol, 2,3-Diethyl-5-methylpyrazin, Essigsäure, Vanillin, 2-Ethyl-3,5-dimethylpyrazin, 2-Methylbutansäure, 3-Methylbutansäure, 3-Methyl-2,4-Nonandion, 2-Methoxyphenol, 2-Phenylethanol, Eugenol und Sotolon.

5. Verfahren zur Herstellung einer Pulverformulierung mit Tabakaroma, das Verfahren umfassend die Schritte:
Vorbereiten eines Tabakausgangsmaterials;
Erwärmen des Tabakausgangsmaterials bei einer Extraktionstemperatur zwischen 100 Grad Celsius und 160 Grad Celsius für wenigstens 90 Minuten;
Auffangen der flüchtigen Verbindungen, die während des Erwärmens aus dem Tabakausgangsmaterial freigesetzt werden;
Bilden einer Tabakaromazusammensetzung, die die aufgefangenen flüchtigen Verbindungen umfasst;
Kombinieren eines Basismaterials und der flüssigen Tabakaromazusammensetzung zum Bilden der Partikel mit Tabakaroma,
wobei in dem Schritt der Vorbereitung des Tabakausgangsmaterials das Tabakausgangsmaterial keiner Behandlung unterzogen wird, die zum Verändern des pH-Wertes des Tabaks geeignet ist.

6. Verfahren nach Anspruch 5, wobei das Tabakausgangsmaterial auf eine Extraktionstemperatur zwischen 120 Grad Celsius und 140 Grad Celsius erwärmt wird.

7. Verfahren nach Anspruch 5 oder 6, wobei das Tabakausgangsmaterial für wenigstens 120 Minuten auf die Extraktionstemperatur erwärmt wird.

8. Verfahren nach einem beliebigen der Ansprüche 5 bis 7, wobei die Extraktionstemperatur so gewählt wird, dass ein Gewichtsverhältnis von (β-Ionon + β-Damascenon) zu (Phenol) in der Tabakaromazusammensetzung von wenigstens etwa 0,25 bereitgestellt wird.

9. Verfahren nach einem beliebigen der Ansprüche 5 bis 8, wobei die Extraktionstemperatur so gewählt wird, dass ein Gewichtsverhältnis von (β-Ionon + β-Damascenon) zu (4-(Methylnitrosamino)-1-(3-pyridyl)-1-butanon + (R, S)-N-Nitrosoanatabin + (R,S)-N-Nitrosoanabasin + N-Nitrosonornicotin + ((2-Furanemethanol)/600)) in der Tabakaromazusammensetzung von wenigstens etwa 1,5 bereitgestellt wird.

10. Verfahren nach einem beliebigen der Ansprüche 5 bis 9, wobei die Extraktionstemperatur so gewählt wird, dass ein Gewichtsverhältnis von (Furanol + (2,3-Diethyl-5-methylpyrazin)*100)) zu (Nikotin) in der Tabakaromazusammensetzung von wenigstens etwa 5 × 10⁻⁴ bereitgestellt wird.

11. Verfahren nach einem beliebigen der Ansprüche 5 bis 10, wobei das Basismaterial ein oder mehrere von einem Gummi, einer Stärke, einer hydrolysierten Stärke, einer chemisch modifizierten Stärke, Carboxymethylcellulose, einem Monosaccharid, einem Disaccharid umfasst.

12. Verfahren nach einem beliebigen der Ansprüche 5 bis 11, wobei der Schritt des Sammelns der flüchtigen Verbindungen, die während des Erwärmens aus dem Tabakausgangsmaterial freigesetzt werden, das Veranlassen der Kondensation der flüchtigen Verbindungen durch Kühlung umfasst.

13. Verfahren nach einem beliebigen der Ansprüche 5 bis 12, wobei der Schritt des Kombinierens des Basismaterials und der flüssigen Tabakaromazusammensetzung zum Bilden von Partikeln mit Tabakaroma umfasst:
Bilden einer Mischung aus dem Basismaterial und der flüssigen Tabakaromazusammensetzung;
Gefrieren der Mischung;
Trocknen der gefrorenen Mischung; und
Mahlen der getrockneten Mischung zum Bilden der Partikel mit Tabakaroma;
oder wobei der Schritt des Kombinierens des Basismaterials und der flüssigen Tabakaromazusammensetzung zum Bilden von Partikeln mit Tabakaroma umfasst:
Bilden einer Mischung aus dem Basismaterial und der flüssigen Tabakaromazusammensetzung;
Sprühtrocknen der Mischung zum Bilden der Partikel mit Tabakaroma.

14. Pulversystem, umfassend:
eine erste Vielzahl von Partikeln nach einem der Ansprüche 1 bis 4, die eine Partikelgröße von wenigstens etwa 20 Mikrometer aufweisen; und
eine zweite Vielzahl von Partikeln, die eine Partikelgröße von etwa 10 Mikrometer oder weniger aufweisen und Nikotin umfassen.

15. Pulversystem, umfassend:
eine erste Vielzahl von Partikeln mit Tabakaroma, die eine Partikelgröße von wenigstens etwa 20 Mikrometer aufweisen, und eine zweite Vielzahl von Partikeln, die eine Partikelgröße von weniger als etwa 20 Mikrometer aufweisen, wobei ein erstes Gewichtsverhältnis von (β-Ionon + β-Damascenon) zu (Phenol) in dem Tabakaroma der ersten Vielzahl größer als 0,25 ist.

## Revendications

1. Formulation de poudre sèche aromatisée de tabac comprenant une pluralité de particules comprenant un matériau de base et une composition aromatisante de tabac, dans laquelle un premier rapport en poids entre (β-ionone + β-damascénone) et (phénol) dans la formulation de poudre sèche aromatisée de tabac est supérieur à 0,25.

2. Formulation de poudre aromatisée de tabac selon la revendication 1 dans laquelle le rapport en poids entre (β-ionone + β-damascénone) et (phénol) dans la formulation de poudre sèche aromatisée de tabac est supérieur à 0,5.

3. Formulation de poudre aromatisée de tabac selon la revendication 1 ou 2 dans laquelle un rapport en poids entre (β-ionone + β-damascénone) et (4-(méthylnitrosamino)-1-(3-pyridyl)-1-butanone + (R,S)-N-nitrosoanatabine + (R,S)-N-nitrosoanabasine + N-nitrosonornicotine + ((2-furaneméthanol)/600)) est supérieur à 1,5.

4. Formulation de poudre aromatisée de tabac selon l'une quelconque des revendications précédentes comprenant en outre un ou plusieurs parmi le furanéol, la 2,3-diéthyl-5-méthylpyrazine, l'acide acétique, la vanilline, la 2-éthyl-3,5-diméthylpyrazine, l'acide 2-méthylbutanoïque, l'acide 3-méthylbutanoïque, la 3-méthyl-2,4-nonanedione, le 2-méthoxyphénol, le 2-phényléthanol, l'eugénol et la sotolone.

5. Procédé de production d'une formulation de poudre aromatisée de tabac, le procédé comprenant les étapes consistant à :
préparer un matériau de départ de tabac ;
chauffer le matériau de départ de tabac à une température d'extraction entre 100 degrés Celsius et 160 degrés Celsius pendant au moins 90 minutes ;
collecter les composés volatils dégagés à partir du matériau de départ de tabac pendant l'étape de chauffage ;
former une composition aromatisante de tabac liquide comprenant les composés volatils collectés ;
combiner un matériau de base et la composition aromatisante de tabac liquide pour former des particules aromatisées de tabac,
dans lequel à l'étape consistant à préparer le matériau de départ de tabac, le matériau de départ de tabac n'est soumis à aucun traitement adapté pour modifier le pH du tabac.

6. Procédé selon la revendication 5, dans lequel le matériau de départ de tabac est chauffé à une température d'extraction entre 120 degrés Celsius et 140 degrés Celsius.

7. Procédé selon la revendication 5 ou 6, dans lequel le matériau de départ de tabac est chauffé à la température d'extraction pendant au moins 120 minutes.

8. Procédé selon l'une quelconque des revendications 5 à 7, dans lequel la température d'extraction est choisie pour fournir un rapport en poids entre (β-ionone + β-damascénone) et (phénol) dans la composition aromatisante de tabac d'au moins environ 0,25.

9. Procédé selon l'une quelconque des revendications 5 à 8, dans lequel la température d'extraction est choisie pour fournir un rapport en poids entre (β-ionone + β-damascénone) et (4-(méthylnitrosamino)-1-(3-pyridyl)-1-butanone + (R,S)-N-nitrosoanatabine + (R,S)-N-nitrosoanabasine + N-nitrosonornicotine + ((2-furaneméthanol)/600)) dans la composition aromatisante de tabac d'au moins environ 1,5.

10. Procédé selon l'une quelconque des revendications 5 à 9, dans lequel la température d'extraction est choisie pour fournir un rapport en poids entre (furanéol + (2,3-diéthyl-5-méthylpyrazine)*100)) et (nicotine) dans la composition aromatisante de tabac d'au moins environ 5 × 10⁻⁴.

11. Procédé selon l'une quelconque des revendications 5 à 10, dans lequel le matériau de base comprend un ou plusieurs parmi une gomme, un amidon, un amidon hydrolysé, un amidon modifié chimiquement, de la carboxyméthylcellulose, un monosaccharide, un disaccharide.

12. Procédé selon l'une quelconque des revendications 5 à 11, dans lequel l'étape consistant à collecter les composés volatils dégagés par le matériau de départ de tabac pendant l'étape de chauffage comprend la condensation des composés volatils par réfrigération.

13. Procédé selon l'une quelconque des revendications 5 à 12 dans lequel l'étape consistant à combiner le matériau de base et la composition aromatisante de tabac liquide pour former des particules aromatisées de tabac comprend :
la formation d'un mélange du matériau de base et de la composition aromatisante de tabac liquide ;
la congélation du mélange ;
le séchage du mélange congelé ; et
le broyage du mélange séché pour former les particules aromatisées de tabac ;
ou dans lequel l'étape consistant à combiner le matériau de base et la composition aromatisante de tabac liquide pour former des particules aromatisées de tabac comprend :
la formation d'un mélange du matériau de base et de la composition aromatisante de tabac liquide ;
le séchage par pulvérisation du mélange pour former les particules aromatisées de tabac.

14. Système de poudre comprenant :
une première pluralité de particules selon l'une quelconque des revendications 1 à 4 et ayant une taille de particule d'au moins environ 20 micromètres ; et
une deuxième pluralité de particules ayant une taille de particule d'environ 10 micromètres ou moins et comprenant de la nicotine.

15. Système de poudre comprenant :
une première pluralité de particules aromatisées de tabac ayant une taille de particule d'au moins environ 20 micromètres et une deuxième pluralité de particules ayant une taille de particule inférieure à environ 20 micromètres, dans lequel un premier rapport en poids entre (β-ionone + β-damascénone) et (phénol) dans le tabac aromatisé de la première pluralité est supérieur à 0,25.
